# EUROPEAN PATENT SPECIFICATION

(11) **EP 2 174 666 B1**
(45) Date of publication and mention of the grant of the patent: **18.12.2013**
(21) Application number: 08765314.3
(22) Date of filing: 09.06.2008
(51) Int. Cl.: A61K 31/41, A61K 31/4178, A61K 31/4184, A61K 31/454, A61P 25/28, A61P 43/00

(54) **MEDICINAL AGENT FOR TREATMENT OF AMYLOID BETA-INDUCED CEREBRAL CIRCULATION OR CEREBRAL BLOOD FLOW DISORDERS**
MEDIZINISCHES MITTEL ZUR BEHANDLUNG VON AMYLOID BETA-INDUZIERTEN STÖRUNGEN DER CEREBRALEN ZIRKULATION ODER DES CEREBRALEN BLUTFLUSSES
AGENT MÉDICAMENTEUX POUR LE TRAITEMENT DE TROUBLES DE LA CIRCULATION CÉRÉBRALE OU DU FLUX SANGUIN CÉRÉBRAL INDUITS PAR L'AMYLOÏDE BÊTA

(30) Priority: 22.06.2007 JP 2007165071
(43) Date of publication of application: 14.04.2010
(73) Proprietor: Daiichi Sankyo Company, Limited, Chuo-ku Tokyo 103-8426 (JP); Osaka University, Suita-shi Osaka 565-0871 (JP)
(72) Inventor: MORISHITA, Ryuichi, Suita-shi Osaka 565-0871 (JP); SATO, Naoyuki, Suita-shi Osaka 565-0871 (JP); TAKEDA, Shuko, Suita-shi Osaka 565-0871 (JP)
(74) Representative: Wallace, Sheila Jane
(86) International application number: PCT/JP2008/060513
(87) International publication number: WO 2009/001661

(56) References cited:
- WO-A2-2007/112288
- JP-A- 05 078 328
- KEHOE ET AL: "Is inhibition of the renin-angiotensin system a new treatment option for Alzheimer's disease?" LANCET NEUROLOGY, LANCET PUBLISHING GROUP, LONDON, GB LNKD- DOI:10.1016/S1474-4422(07)70077-7, vol. 6, no. 4, 1 April 2007 (2007-04-01), pages 373-378, XP005931881 ISSN: 1474-4422
- TSUKUDA K. ET AL.: 'Koshokuen.Koshiboshoku Fuka de Mitomerareta Ninchi Kino no Teika ni Taisuru Olmesartan to Azelnidipine no Koka' JAPANESE JOURNAL OF GERIATRICS vol. 44, 20 May 2007, page 129, XP008127986
- GARD P.R.: 'Angiotensin as a target for the treatment of Alzheimer's disease, anxiety and depression' EXPERT OPIN. THER. TARGETS vol. 8, no. 1, 2004, pages 7 - 14, XP009078785
- ROSENDORFF C.: 'Certain anti-hypertensive agents reduce beta-amyloid neuropathology and improve cognition in a mouse model of Alzheimer's disease' CIRCULATION vol. 114, no. 18, 2006, page 129, XP008128003
- TRENKWALDER P.: 'Potential for antihypertensive treatment with an AT(1)-receptor blocker to reduce dementia in the elderly' J. HUM. HYPERTENS. vol. 16, no. SUPPL. 3, 2002, pages S71 - S75, XP008128004
- IWAI M. ET AL.: 'The calcium-channel blocker, azelnidipine, enhances the inhibitory action of AT1 receptor blockade on ischemic brain damage' J. HYPERTENS. vol. 24, no. 10, 2006, pages 2023 - 2031, XP008128005
- HOSOMI N. ET AL.: 'Angiotensin type 1 receptor blockage improves ischemic injury following transient focal cerebral ischemia' NEUROSCIENCE vol. 134, no. 1, 2005, pages 225 - 231, XP004971926
- GIROUARD H. ET AL.: 'Angiotensin II attenuates endothelium-dependent responses in the cerebral microcirculation through nox-2-derived radicals' ARTERIOSCLER. THROMB. VASC. BIOL. vol. 26, no. 4, 2006, pages 826 - 832, XP008128002
- MOGI M. ET AL.: 'Inhibition of cognitive decline in mice fed ad high-salt and cholesterol diet by the angiotensin receptor blockade, olmesartan' NEUROPHARMACOLOGY vol. 53, no. 8, 2007, pages 899 - 905, XP022358182
- WANG J. ET AL.: 'Valsartan lowers brain beta-amyloid protein levels and improves spatial learning in a mouse model of Alzheimer disease' J. CLIN. INVEST. vol. 117, no. 11, 2007, pages 3393 - 3402, XP002589379

## Description

### TECHNICAL FIELD

The present invention relates to a medicament for treating, amyloid β-induced cerebral circulation or cerebral blood flow disorders comprising an angiotensin II receptor blocker as an active ingredient.

### BACKGROUND ART

No fundamental treatment protocol for Alzheimer's disease has been developed yet. Only cholinesterase inhibitor and the like are used palliatively. Besides, medicaments which seem to be effective in preventing Alzheimer's disease have not been found yet.

Recently, it has become clear that vascular risk factors are deeply involved in the background pathology of not only vascular dementia but also Alzheimer's disease; among all, hypertension is regarded as a crucial factor (Non-Patent Document 1). Some clinical tests have suggested that a part of calcium antagonist and angiotensin-converting enzyme inhibitor (both are antihypertensive drugs) may be capable of inhibiting the onset of Alzheimer's disease (Non-Patent Document 2). With respect to angiotensin II receptor blocker (ARB), no report of its efficacy for Alzheimer's disease has been made yet.
Non-Patent Document 1: Launer LJ et al., Neurology 52:78, 1999
Non-Patent Document 2: Forette F et al., Lancet 352:1347, 1998

Tsukuda K et al., Japanese Journal of Geriatrics, vol.44,20 May 2007, 129 reported the effects of administering the ARB olmesartan to mice fed on a high-salt, high-fat diet, and concluded that olmesartan suppressed cognitive decline due to a high-salt, high-fat diet.

WO 2007/112288 published 4 October 2007 discloses olmesartan medoxomil for use in reducing Aβ1-40 and Aβ1-42 generation.

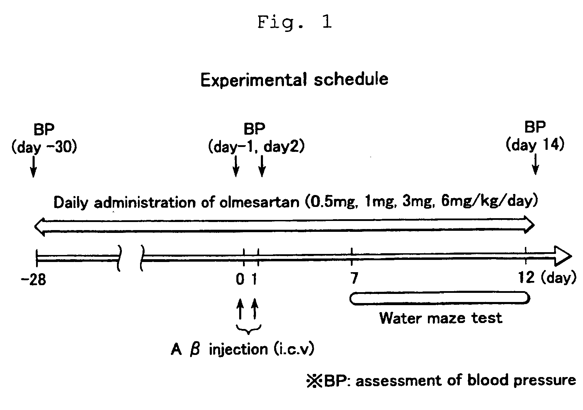

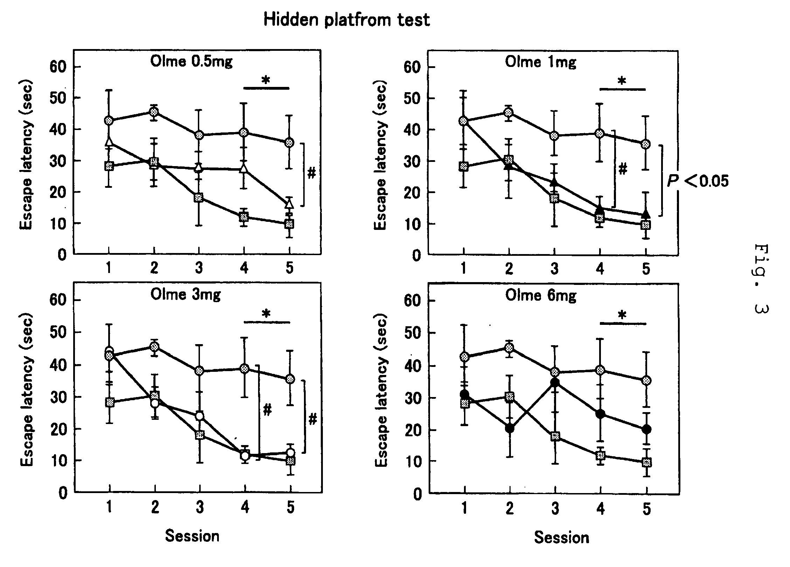

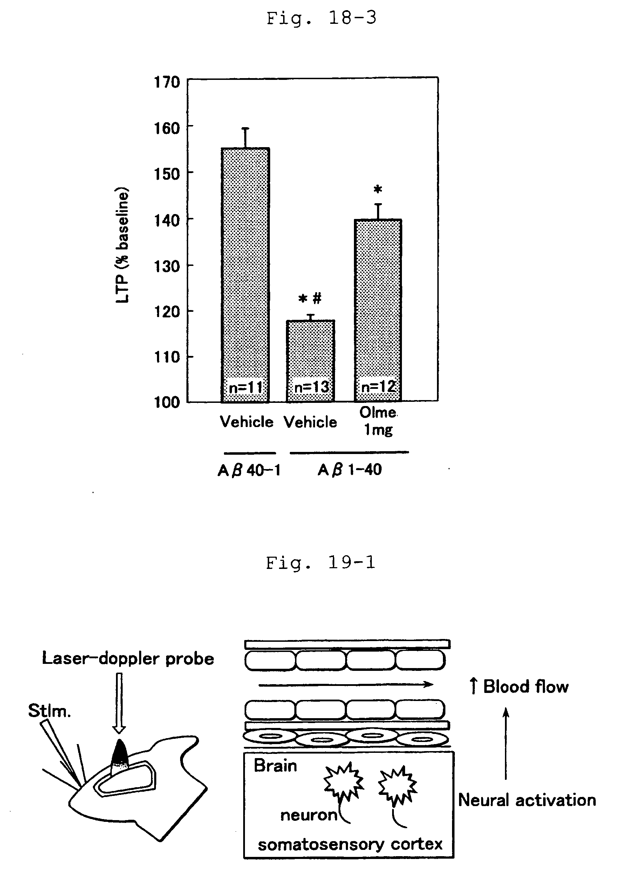

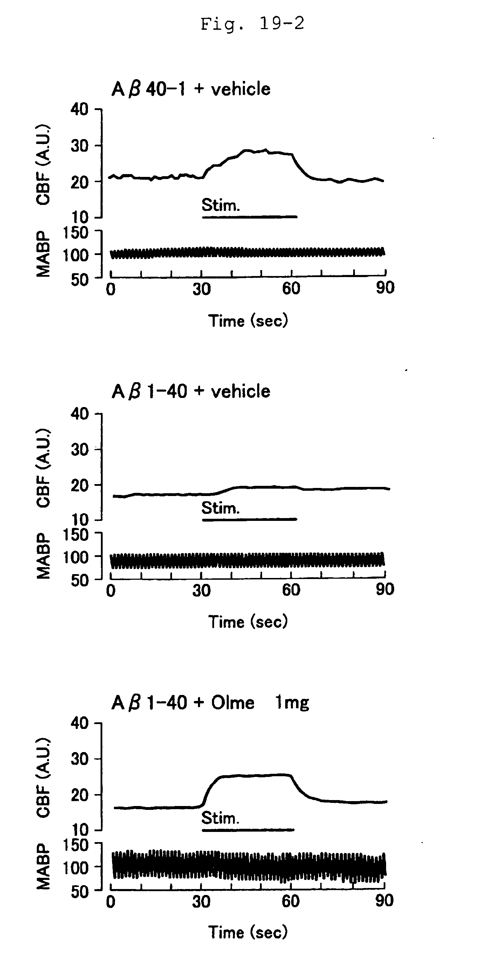

The present inventors examined the therapeutic effect of orally administered olmesartan using a mouse model called "Aβ injection model" which initiates cognitive impairment upon acute administration of amyloid β (a probable causative agent of Alzheimer's disease) to the lateral ventricle (Fig. 1). For the assessment of cognitive function, "Morris water maze test" which evaluates spatial reference memory was used. In those mice which had received oral administration of olmesartan for four weeks in advance, the cognitive impairment seen in this model was ameliorated (Figs. 3 to 6). It was suggested that this effect is especially great at the dose level of olmesartan 1 mg/kg/day or less at which no excessive lowering in blood pressure occurs (Figs. 3 to 6). As to the mechanism of this therapeutic effect, the inventors believe that amyloid β-impaired cerebrovascular activity was improved (probably because of the antioxidative effect, etc. of olmesartan) and that improved cerebral circulation enhanced the excretion of amyloid β from the brain (Figs. 19 to 21).

The present invention may be summarized as follows.
(1) 4-(1-Hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methylimidazole-5-carboxylate for use in the treatment of amyloid β-induced cerebral circulation or cerebral blood flow disorders.
(2) A composition comprising 4-(1-hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methyl-imidazole-5-carboxylate as an active ingredient, for use in the treatment of amyloid β-induced cerebral circulation or cerebral blood flow disorders.
(3) Use of 4-(1-hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methylimidazole-5-carboxylate for the manufacture of a medicament for the treatment of amyloid β-induce cerebral circulation or cerebral blood flow disorders.

The present inventors have demonstrated a possibility that olmesartan, a type of angiotensin II receptor blocker, may improve the cognitive impairment of an Alzheimer's disease model mouse. Besides, since this drug (olmesartan) has been used clinically as an antihypertensive drug, it is remarkably characteristic that Alzheimer's disease patients will have little problem in terms of safety in receiving oral administration of this drug.

### EFFECT OF THE INVENTION

According to the present invention, it becomes possible to provide a medicament useful in treating amyloid β-induced cerebral circulation or cerebral blood flow disorders.

### BRIEF DESCRIPTION OF THE DRAWINGS

Fig. 1 shows the experimental schedule of Test Example 1.
Fig. 2 shows changes in blood pressure caused by administration of olmesartan in Test Example 1.
Fig. 3 shows the results of hidden platform test in Test Example 1.
Fig. 4 shows the results of visible platform test in Test Example 1.
Fig. 5 shows the results of probe test in Test Example 1.
Fig. 6 shows the results of annulus crossing in Test Example 1.
Fig. 7 shows the experimental schedule of Test Example 2.
Fig. 8 shows changes in blood pressure caused by administration of olmesartan or hydralazine in Test Example 2.
Fig. 9 shows the results of hidden platform test in Test Example 2.
Fig. 10 shows the results of visible platform test in Test Example 2.
Fig. 11 shows the results of probe test in Test Example 2.
Fig. 12 shows the results of annulus crossing in Test Example 2.
Fig. 13 shows the swimming speed of each group in Test Example 2.

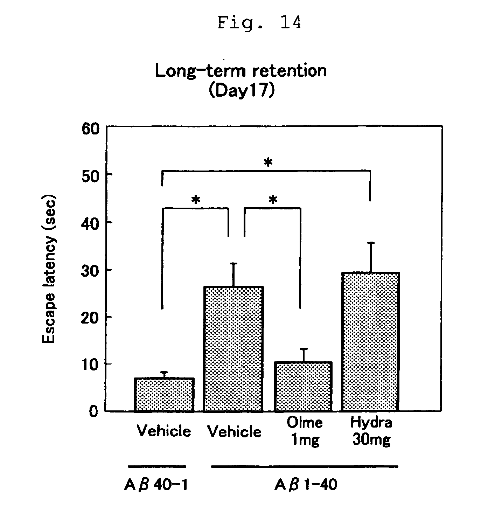

Fig. 14 shows the results of long-term retention in Test Example 2.
Fig. 15 shows the results of working memory task in Test Example 2.
Fig. 16 shows the results of open field test in Test Example 2.
Fig. 17 is a pie graph showing the percentages of various causative diseases for dementia.
Fig. 18-1 shows the response traces of field EPSP before and 60 min after tetanic stimulation in Test Example 3.
Fig. 18-2 shows the averaged time course of field potential slope values in Test Example 3.
Fig. 18-3 shows field potential slope values taken 50-60 min after tetanic stimulation in Test Example 3.
Fig. 19-1 is a schematic diagram showing that neuronal activity is evoked in the somatosensory-cortex by whisker stimulation performed in Test Example 3.
Fig. 19-2 shows the CBF response to the whisker stimulation in Test Example 3.
Fig. 20-1 is a schematic diagram showing the experimental method for the monitoring of cerebral blood flow in Test Example 3 (overall view).
Fig. 20-2 is a schematic diagram showing the experimental method for the monitoring of cerebral blood flow in Test Example 3 (enlarged view of the brain).
Fig. 20-3 shows the relation between CBF and MABP in Test Example 3.
Fig. 21 shows the mechanism of action of olmesartan as presumed from the results of Test Example 3.

Preferably, the angiotensin II receptor blocker is olmesartan medoxomil. In particular, olmesartan medoxomil may be prepared easily according to the method disclosed, for example, in Japanese Patent No. 2082519 (United States Patent No. 5,616,599).

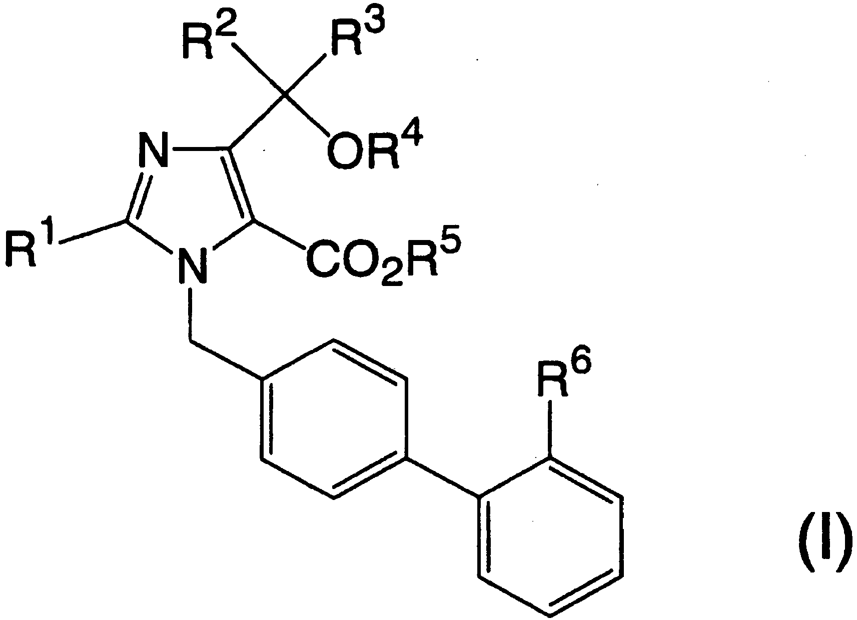

**Table 1**

| Compound No. | R¹ | R² | R³ | R⁴ | R⁵ | R⁶ |
|---|---|---|---|---|---|---|
| 29 | Pr | Me | Me | H | H | Tz |
| 37 | Pr | Me | Me | H | Mod | Tz |
| Abbreviations used in the above Table indicate the following groups. Me: methyl group Mod: (5-methyl-2-oxo-1,3-dioxolen-4-yl)methyl group Pr: propyl group Tz: tetrazol-5-yl group | | | | | | |

Illustrated compound No. 29: 4-(1-hydroxy-1-methylethyl)-2-propyl-1-[4-[2-(tetrazol-5-yl)phenyl]phenyl]methylimidazole-5-carboxylic acid (Japanese designation: olmesartan), and

Illustrated compound No. 37: (5-methyl-2-oxo-1,3-dioxolen-4-yl)methyl 4-(1-hydroxy-1-methylethyl)-2-propyl-1-[4-[2-(tetrazol-5-yl)phenyl]phenyl]methyl-imidazole-5-c arboxylate (Japanese designation: olmesartan medoxomil).

Olmesartan medoxomil (illustrated compound No. 37) is a prodrug of olmesartan (illustrated compound No. 29), in which the carboxylic acid at position 5 of the imidazole ring is medoxomil ester. Upon oral administration, this prodrug is hydrolyzed by esterase, mainly in the small intestinal epithelium, and thus converted to the active form olmesartan.

The compound (I) which is the active ingredient of the present invention, pharmacologically acceptable salts thereof and pharmacologically acceptable esters thereof are known (see, for example, Japanese Unexamined Patent Publication No. Hei 5-78328) or they may be prepared by known methods (see, for example, Japanese Unexamined Patent Publication No. Hei 5-78328).

According to the present invention the medicament of the present invention is used to improve amyloid β-induced cerebral circulation or cerebral blood flow disorder. Further, the medicament of the present invention may be used in the treatment of amyloid β-induced brain dysfunction including Alzheimer is disease.

Alzheimer's disease is a neurodegenerative disease. A characteristic pathological finding called senile plaques is observed in autopsy brain specimen from patients with Alzheimer's disease. It is known that the major component of these senile plaques is a protein called amyloid β (Aβ), and it is presumed that Aβ is a causative agent for Alzheimer's disease. Recently, epidemiologic studies have shown that the presence of hypertension in middle-aged people is an independent risk factor for Alzheimer's disease, and large-scale clinical tests revealed that antihypertensive therapy reduces the risk of developing Alzheimer's disease. Thus, a possibility has been suggested that hypertension is deeply involved in the background pathology of Alzheimer's disease.

As shown in Examples described later, the medicament of the present invention is capable of improving the dysfunction/impairment which amyloid β induces, e.g., impairment of spatial memory and cerebrovascular disorders such as cerebral circulation/cerebral blood flow disorders. Further, the dysfunction/impairment-improving effect of the medicament of the present invention is not limited to its action on amyloid β-induced dysfunction/impairment.

Since the above-described angiotensin II receptor blocker is generally administered orally, it is desirable to administer the medicament of the present invention orally. However, the administration route of the medicament of the present invention is not limited to oral administration. It may also be administered parenterally, such as intravenously, intrarectally, percutaneously, transmucosally or subcutaneously. Examples of dosage forms suitable for oral administration include, but are not limited to, powder, granules, tablets and capsules. In the preparation of unit dosage form, pharmacologically acceptable additives for formulation such as excipients, lubricants, binders, disintegrants, emulsifiers, stabilizers, correctives or diluents may be used appropriately.

Examples of "excipients" that may be used include: organic excipients including sugar derivatives such as lactose, sucrose, glucose, mannitol or sorbitol; starch derivatives such as corn starch, potato starch, α-starch or dextrin; cellulose derivatives such as microcrystalline cellulose; gum arabic; dextran; and pullulan; and inorganic excipients including silicate derivatives such as light silicic acid anhydride, synthetic aluminium silicate, calcium silicate or magnesium aluminometasilicate; phosphates such as calcium hydrogenphosphate; carbonates such as calcium carbonate; and sulfates such as calcium sulfate.

Examples of "lubricants" that may be used include: stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate or magnesium lauryl sulfate; silicates such as silicic anhydride and silicic hydrate; and the starch derivatives described above.

Examples of "binders" that may be used include: hydroxypropylcellulose, hydroxypropylmethylcellulose, polyvinylpyrrolidone, macrogol, and compounds similar to the above-described excipients.

Examples of "disintegrants" that may be used include: cellulose derivatives such as low-substituted hydroxypropylcellulose, carboxymethylcellulose, carboxymethylcellulose calcium or internally crosslinked sodium carboxymethylcellulose; crosslinked polyvinylpyrrolidone; and chemically modified starch/cellulose derivatives such as carboxymethylstarch or carboxymethylstarch sodium.

Examples of "emulsifiers" that may be used include: colloidal clay such as bentonite or veegum; metal hydroxides such as magnesium hydroxide or aluminium hydroxide; anionic surfactants such as sodium lauryl sulfate or calcium stearate; cationic surfactants such as benzalkonium chloride; and nonionic surfactants such as polyoxyethylenealkylether, polyoxyethylene sorbitan fatty acid ester or sucrose esters of fatty acids.

Examples of "stabilizers" that may be used include: p-hydroxybenzoate esters such as methylparaben or propylparaben; alcohols such as chlorobutanol, benzyl alcohol or phenylethyl alcohol; benzalkonium chloride; phenols such as phenol or cresol; thimerosal; dehydroacetic acid; and sorbic acid.

Examples of "correctives" that may be used include: sweeteners such as saccharin sodium or aspartame; acidifiers such as citric acid, malic acid or tartaric acid; and flavors such as menthol, lemon or orange.

Examples of "diluents" that may be used include conventionally used diluents, such as, lactose, mannitol, glucose, sucrose, calcium sulfate, calcium phosphate, hydroxypropylcellulose, microcrystalline cellulose, water, ethanol, polyethylene glycol, propylene glycol, glycerol, starch, polyvinylpyrrolidone, magnesium aluminometasilicate, and mixtures of these compounds.

Doses of the medicament of the present invention may be appropriately selected depending on various factors such as the administration route, the type of the active ingredient, the age, body weight or symptoms of the patient, the purpose of administration, etc. Generally, the medicament of the present invention is administered at a level within a range from the lower limit of 0.08 mg/kg (preferably 0.33 mg/kg, more preferably 0.50 mg/kg) per day to the upper limit of 1.13 mg/kg (preferably 1.0 mg/kg) per day. This dose may be administered once or 2 to 6 times a day depending on the symptoms.

The medicament of the present invention may be used in combination with other preparations that are effective against the target disease (e.g., Alzheimer's disease). For example, donepezil hydrochloride effective for Alzheimer's disease may be used jointly.

### EXAMPLES

Hereinbelow, the present invention will be described in more detail with reference to Test Examples and Preparation Examples. However, the scope of the present invention is not limited to these Examples.

### Materials

Amyloid β (Aβ) 1-40 and Aβ 40-1 (Peptide Institute, Inc.)
* Aβ 40-1 has a sequence reverse to that of Aβ 1-40. This was used as a control peptide. ddY mouse (CLEA Japan, Inc.)

Olmesartan medoxomil (Daiichi Sankyo Company, Limited)

Hydralazine (Sigma)

### TEST EXAMPLE 1

### Methods

The therapeutic effect of olmesartan medoxomil in Aβ injection model and the dose dependency thereof were examined according to the following experimental schedule. [Experimental Schedule]: Prior to the experiment, 8-week old male ddY mice were administered olmesartan medoxomil (0.5 mg/kg/day, 1 mg/kg/day, 3 mg/kg/day or 6 mg/kg/day) or distilled water orally for four weeks (day -28 to -1). Then, Aβ 1-40 was administered to the mice intracerebroventricularly (day 0). (Although Aβ 1-40 was administered into one of the lateral ventricles in WO 2006/059777, Aβ 1-40 was administered in Test Example 1 to both lateral ventricles with an interval of one day between two administrations. Except for this point, Aβ 1-40 administration was performed in the same manner as in WO 2006/059777.) From one week thereafter, a behavioral test to evaluate cognitive function (Morris water maze test; Morris R et al., J Neuroscience Methods 1984, 11, 47-60) was performed (day 7 to 12). As a negative control, mice which had received oral administration of distilled water for four weeks were administered Aβ 40-1 intracerebroventricularly. The administration of olmesartan medoxomil or distilled water was continued until the end of the behavioral test. Measurements were performed as described below.

Hidden platform test: A platform is placed below the water surface of a water pool 150 cm in diameter filled with white water-colored. Mice are released into the water, and the time it takes them to reach the platform is measured. This trial is performed three times a day and for five consecutive days. As they learn, mice become able to reach the platform more quickly day by day.

Visible platform test: Mice are released into the water under such conditions that they can see the platform. The time it takes them to reach the platform is measured. Probe test: The platform is removed, and mice are released and allowed to swim freely. Those mice with good learning/memory swim in the quadrant where the platform was located searching for the platform. The time they spent in that quadrant is measured. Annnulus crossing: The number of times mice crossed the platform in the probe test is counted.

Further, assessment of blood pressure was performed by the tail-cuff method (using a sphygmomanometer wound around the mouse tail) (Bunag RD, J Lab Clin Med. 1971, 78, 957-62) immediately before the start of administration of olmesartan medoxomil or distilled water (day -30), immediately before (day -1) and immediately after (day 2) the Aβ intracerebroventricular administration, and after completion of the behavioral test (day 14) (see Fig. 1).

### Results

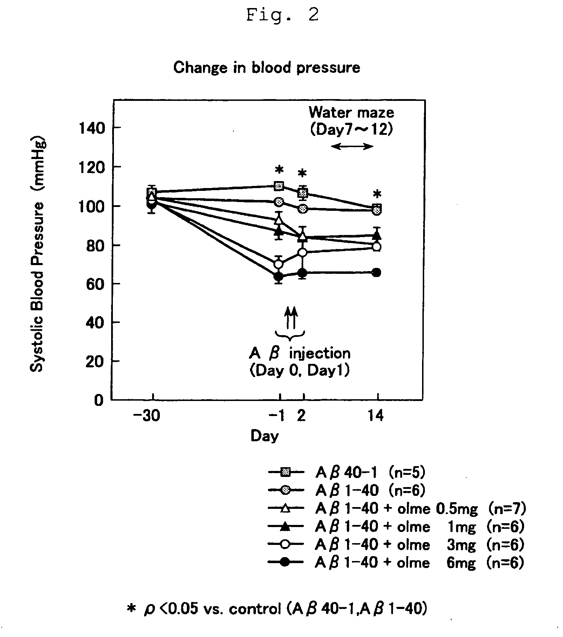

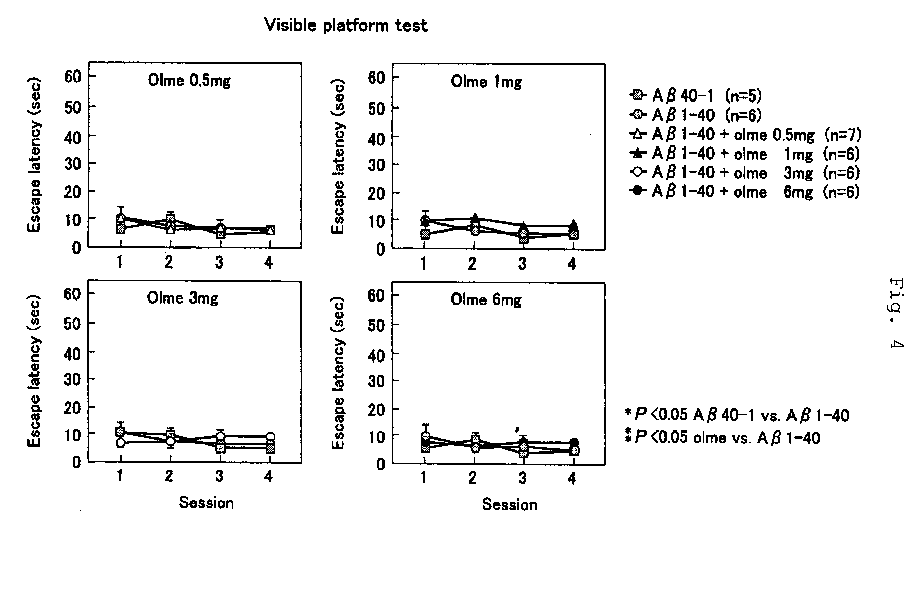

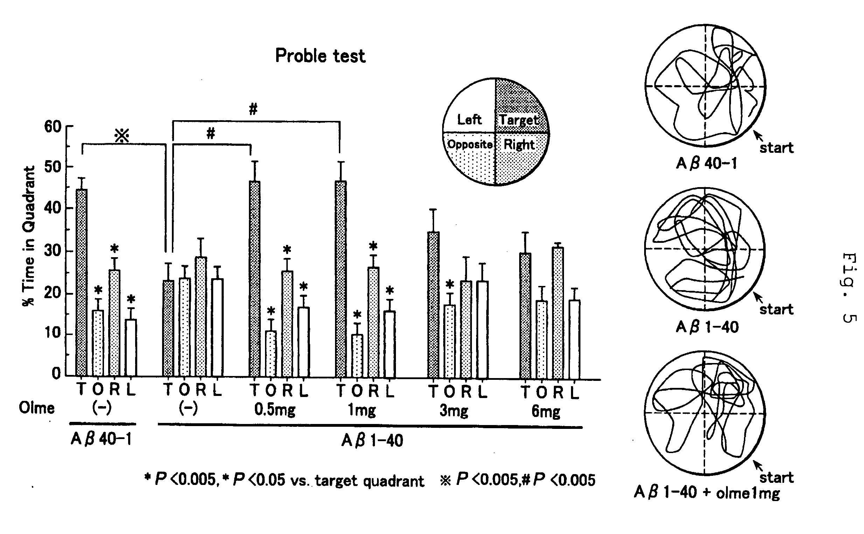

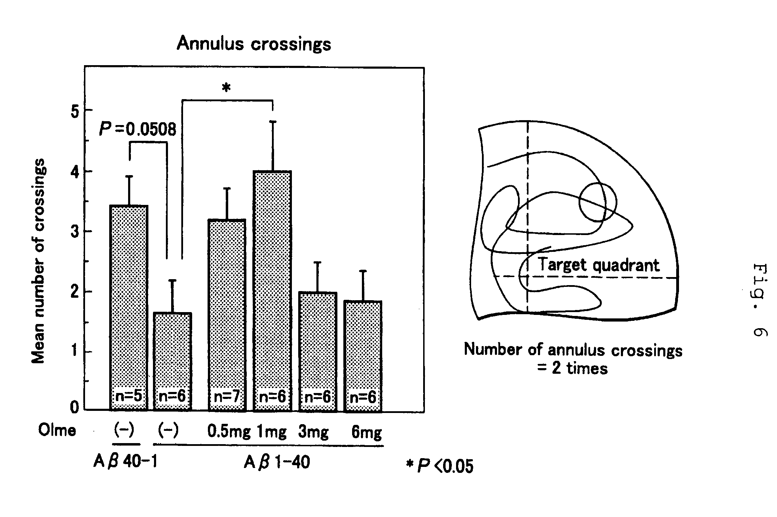

- Blood pressure: No difference between groups was observed in the blood pressure before the start of administration of olmesartan medoxomil or distilled water (day -30). After 4-week administration (day -1), the blood pressures in olmesartan medoxomil administration groups were significantly lowered compared to the distilled water administration group. Even the group with the lowest dose of olmesartan medoxomil (0.5 mg/kg/day) showed a significant lowering in the blood pressure. A similar tendency was observed in the measurements performed immediately after Aβ administration (day 2) and after completion of the behavioral test (day 14) (see Fig. 2).
- Cognitive function: It was confirmed in the hidden platform test that the escape latency was significantly longer in Aβ 1-40 administration group (●) than in Aβ 40-1 administration group (■) on day 4 and 5 after the start of learning (session 4 and 5) and that the former group had impairment in spatial memory and learning ability However, this dysfunction was improved significantly in olmesartan medoxomil 0.5 mg/kg/day administration group (Δ), 1.0 mg/kg/day administration group (A) and 3.0 mg/kg/day administration group (○) (see Fig. 3). In the visible platform test, no significant difference was observed in the escape latency between groups; thus, it was confirmed that there was no difference in confounding factors (see Fig. 4). In the probe test, the time the mice spent in the target quadrant was decreased significantly in Aβ 1-40 administration group (●) compared to Aβ 40-1 administration group (■). However, this was significantly improved in olmesartan medoxomil 0.5 mg/kg/day administration group (Δ) and 1.0 mg/kg/day administration group (▲) (see Fig. 5). The number of annulus crossings tended to be smaller in Aβ 1-40 administration group (●) than in the Aβ 40-1 administration group (■), but this was significantly improved in olmesartan medoxomil 1.0 mg/kg/day administration group (▲) (see Fig. 6). From these results, it was confirmed that in the Morris water maze test, the impairment in spatial memory and learning evoked by intracerebroventricular administration of Aβ 1-40 was significantly improved in olmesartan medoxomil administration groups. It can be said that this effect was observed in low olmesartan medoxomil dose groups (0.5- 1 mg/kg/day) but that it was not observed in high dose groups (3-6 mg/kg/day).

### TEST EXAMPLE 2

### Methods

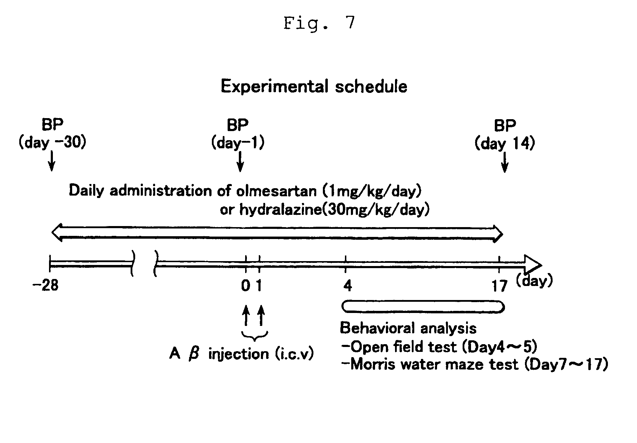

In order to examine whether the improvement effect on cognitive function achieved by oral administration of olmesartan medoxomil (Test Example 1) was caused by lowered blood pressure *per se* or not, a similar experiment was performed using hydralazine administration group as a hypotensive control (see Fig. 7). The dose of olmesartan medoxomil was set at a low level of 1 mg/kg/day in view of the experimental results of Test Example 1. On the other hand, it was confirmed from the results of a pilot experiment that the dose of hydralazine which produces a comparable level of blood pressure lowering is 30 mg/kg/day. As behavioral tests to assess cognitive function, Morris water maze tests were performed as in Test Example 1 (hidden platform test, visible platform test and probe test) and, in addition, an open field test was also performed. Further, long-term retention, working memory task and swimming speed were also examined in the Morris water maze tests. Measurements were performed as described below.

Open field test: A cubic box (30 x 30 x 30 cm) is used. On each X and Y bank of the open-field area, two infrared beams are attached 2 cm above the floor at a 10 cm interval. The number of successive interceptions by the subject of the two adjoining beams on each bank is counted as locomotion score.

Long-term retention: On day 12 after the completion of the hidden platform test as a water maze test, mice are released into the water and the time it takes them to reach the platform is measured.

Working memory task: The platform is transferred and fixed at a new place. Mice are given four consecutive trials at intervals of as short as 20 seconds, and the time it takes them to reach the platform is measured. The average of the escape latency values for the 2nd to 4th trial was taken as an indicator of working memory task.

Swimming speed: The time it takes the mice to reach the platform is affected by the swimming speed of each mouse. In order to eliminate this influence, swimming speed is measured.

### Results

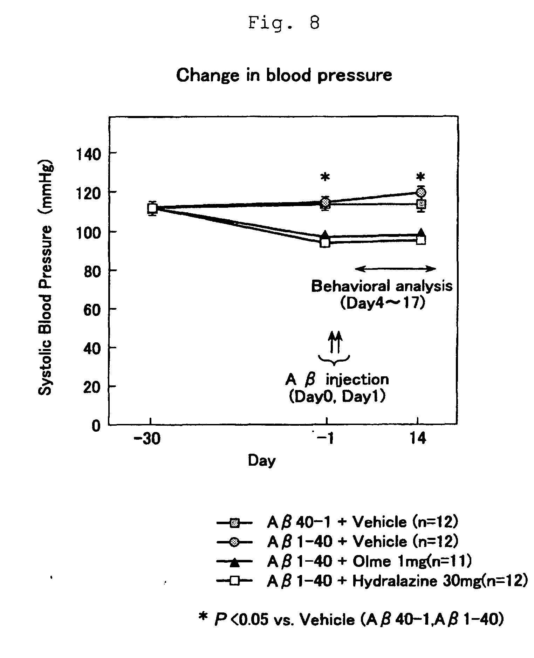

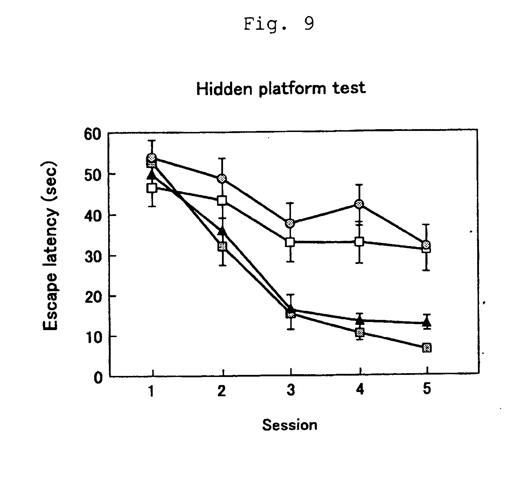

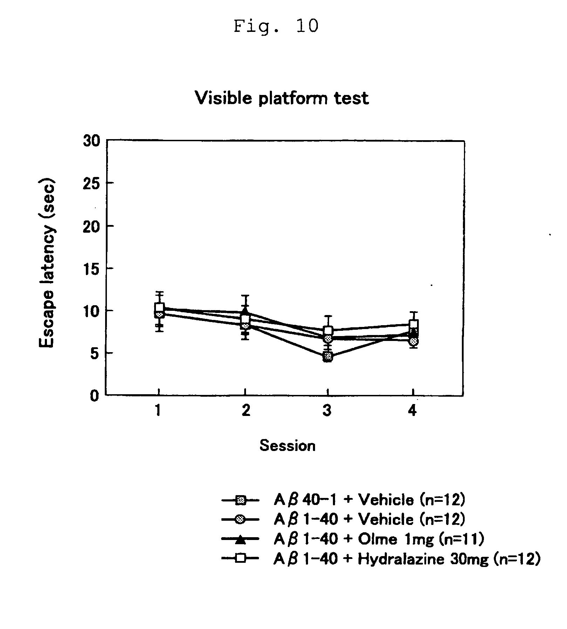

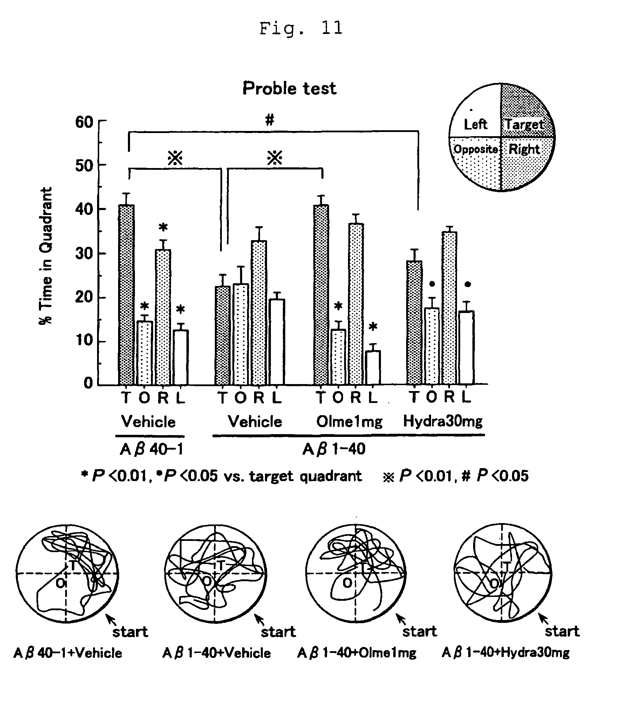

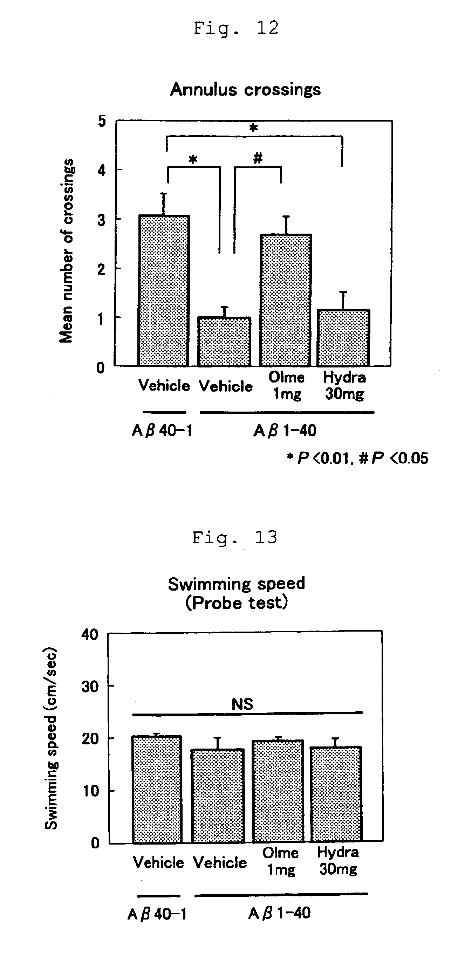

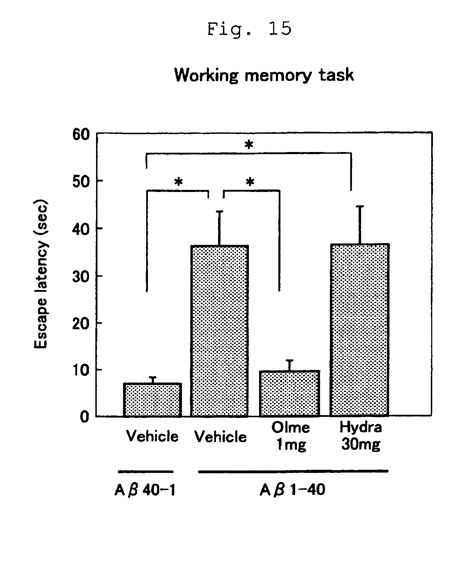

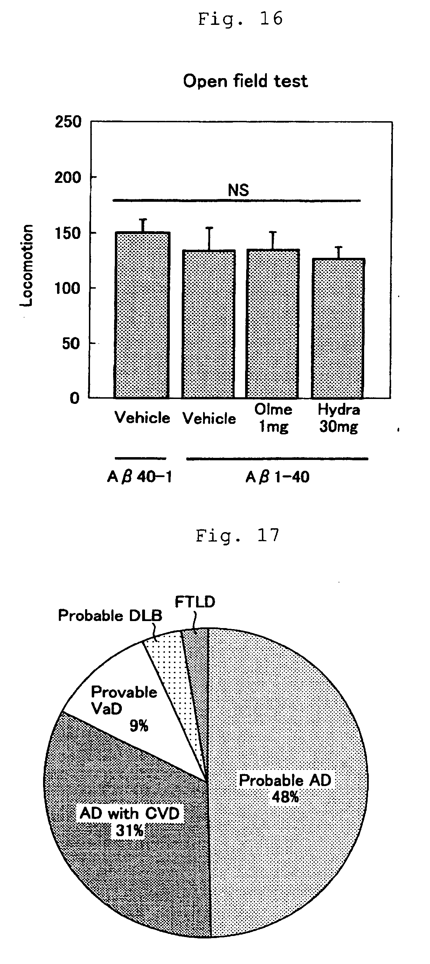

- Blood pressure: No significant difference was observed in blood pressure between groups before the start of oral administration, but after 4-week administration (day -1), it was confirmed that the blood pressures in olmesartan medoxomil 1 mg/kg/day group and hydralazine 30 mg/kg/day group were lowered significantly and by almost equal levels compared to distilled water administration group (see Fig. 8).
- Cognitive function: In the hidden platform test, the time it took the mice to reach the platform was shortened through successive sessions in Aβ 40-1 administration group. However, the time was not shortened in Aβ 1-40 administration group. In Aβ 1-40 + olmesartan medoxomil administration group, the time it took the mice to reach the platform was shortened through successive sessions, as compared to Aβ 40-1 administration group. In Aβ 1-40 + hydralazine administration group, the time was not shortened (Fig. 9).
- Visible platform test: No significant difference was observed between groups (Fig. 10).
- Probe test: The time it took the mice to reach the platform was shortened through successive sessions in Aβ 40-1 administration group. However, the time was not shortened in Aβ 1-40 administration group. In Aβ 1-40 + olmesartan medoxomil administration group, the time it took the mice to reach the platform was shortened through successive sessions, as compared to Aβ 40-1 administration group. In Aβ 1-40 + hydralazine administration group, the time was not shortened (Fig. 11).
- Annulus crossings means the number of times that mice crossed the platform in the probe test. In Aβ 40-1 administration group, the time it took the mice to reach the plat form was shortened. However,, the number of platform crossings was smaller in Aβ 1-40 administration group than in Aβ 40-1 administration group. While the number of platform crossings increased in Aβ 1-40 + olmesartan medoxomil administration group compared to Aβ 1-40 administration group, there was increase in Aβ 1-40 + hydralazine administration group (Fig. 12).
- Swimming speed: No significant difference was observed between groups (Fig. 13).
- Long-term retention: On day 17, the time it took the mice to reach the platform was longer in Aβ 1-40 administration group than in Aβ 40-1 administration group. The time it took the mice to reach the platform was shortened in Aβ 1-40 + olmesartan medoxomil administration group compared to Aβ 1-40 administration group. However, the time it took the mice to reach the platform was not shortened in Aβ 1-40 + hydralazine administration group (Fig. 15).
- Working memory task: The time it took the mice to reach the platform was longer in Aβ 1-40 administration group than in Aβ 40-1 administration group. The time it took the mice to reach the platform was shortened in Aβ 1-40 + olmesartan medoxomil administration group compared to Aβ 1-40 administration group. However, the time it took the mice to reach the platform was not shortened in Aβ 1-40 + hydralazine administration group (Fig. 15).
- Open field test: No significant difference was observed between groups (Fig. 16).

What has been stated so far may be summarized as follows. It was confirmed in the Morris water test that the spatial memory impairment and learning disability evoked by intracerebroventricular administration of Aβ 1-40 were significantly improved in olmesartan medoxomil 1 mg/kg/day group as in Test Example 1, but this effect was not observed in hydralazine 30 mg/kg/day group. It was confirmed in the open field test and the like that there was no considerable difference in confounding factors such as the activity or swimming ability of mice.

Using an Aβ injection model, the present inventors found a possibility that the spatial memory impairment and learning disability evoked by intracerebroventricular administration of Aβ 1-40 may be improved by oral administration of a low dose of olmesartan medoxomil (Test Example 1). Further, by performing a similar experiment using hydralazine as a control drug, the inventors confirmed that this effect was not produced by lowered blood pressure *per se* (Test Example 2). The effect was not observed in olmesartan medoxomil high dose groups (3-6 mg/kg/day), probably because the cerebral blood flow was reduced by excessive lowering of blood pressure (i.e. somatic blood pressure dropped below the lower limit of cerebrovascular autoregulation).

### TEST EXAMPLE 3

### Methods

Electrophysiology: Long term potentiation was measured as previously reported (Niisato K, Fujikawa A, Komai S, Shintani T, Watanabe E, Sakaguchi G, Katsuura G, Manabe T, Noda M: Age-dependent enhancement of hippocampal long-term potentiation and impairment of spatial learning through the Rho-associated kinase pathway in protein tyrosine phosphatase receptor type Z-deficient mice. J Neurosci 2005; 25:1081-1088). Transverse hippocampal slices (400 µm thick) were prepared from 5- to 10-week-old mice or from 13- to 31-week-old mice and placed in an interface-type holding chamber for at least 1 hr. A single slice was then transferred to a recording chamber and submerged beneath a continuously perfusing medium saturated with 95% O₂ and 5% CO₂. The medium was composed of 119 mM NaCl, 2.5 mM KCl, 1.3 mM MgSO₄, 2.5 mM CaCl₂, 1.0 mM NaH₂PO₄, 26.2 mM NaHCO₃ and 11 mM glucose. All of the perfusing solutions contained 100 µM picrotoxin in order to block GABA_{A} receptor-mediated inhibitory synaptic responses. The field potential recordings were made with a glass electrode (3 M NaCl) placed in the stratum radiatum. The whole-cell pipette solution contained 122.5 mM Cs gluconate, 17.5 mM CsCl, 10 mM HEPES, 0.2 mM EGTA, 8 mM NaCl, 2 mM Mg-ATP and 0.3 mM Na₃-GTP, pH 7.2; 290-300 mM mOsm. For evoking synaptic responses, a bipolar tungsten stimulating electrode was placed in the stratum radiatum, and Schaffer collateral/commissural fibers were stimulated at 0.1 Hz. The stimulus strength was adjusted so that it gave rise to AMPA receptor-mediated EPSPs of the slope values between 0.10 and 0.15 mV/ms. For the analysis of EPSPs, the inventors measured their early rising phase in order to avoid contamination by voltage-dependent components as much as possible. Each data point represents the averaged slope value for 1 min that was normalized to the baseline slope value. All experiments were performed at 25°C. The data are expressed as mean ± SEM. Student's t test or ANOVA was used to determine whether there was a significant difference (p < 0.05) in the mean. The majority of electrophysiological experiments were blind tests, and the results were essentially identical to those of the non-blind experiments; thus, all of the data were pooled. Picrotoxin was purchased from Wako (Osaka, Japan). Monitoring of cerebral blood flow: A small craniotomy (2 x 2 mm) was performed to expose the somatosensory cortex; the dura was removed; and the site was perfused with a modified Ringer's solution (37°C; pH 7.3-7.4) (Iadecola C, Zhang F, Niwa K, Eckman C, Turner SK, Fischer E, Younkin S, Borchelt DR, Hsiao KK, Carlson GA: SOD1 rescues cerebral endothelial dysfunction in mice overexpressing amyloid precursor protein. Nat Neurosci 1999; 2:157-161). Cerebral blood flow (CBF) was continuously monitored at the site of perfusion with a laser-Doppler probe positioned stereotaxically on the cortical surface and connected to a computerized data acquisition system. CBF values were expressed as percent increase relative to the resting level. Zero values for CBF were obtained after the heart was stopped by an overdose of isoflurane at the end of the experiment. Effect of topical application of Aβ40 on functional hyperemia: The cranial window was first perfused with Ringer's solution, and CBF responses to whisker stimulation were recorded. The whiskers were gently stroked for 60 sec with a cotton-tipped applicator. After testing CBF responses during Ringer's solution perfusion, the perfusion solution was changed to Ringer's solution containing Aβ40 (5 µM; Peptide Institute, Osaka, Japan). This concentration of Aβ40 was previously found to produce maximal cerebrovascular effects (Niwa K, Younkin L, Ebeling C, Turner SK, Westaway D, Younkin S, Ashe KH, Carlson GA, Iadecola C: Abeta 1-40-related reduction in functional hyperemia in mouse neocortex during somatosensory activation. Proc Natl Acad Sci U S A 2000; 97:9735-9740). Cerebrovascular autoregulation: Techniques used for studying cerebrovascular autoregulation in rodents were similar to those previously described (Ishitsuka T, Iadecola C, Underwood MD, Reis DJ: Lesions of nucleus tractus solitarii globally impair cerebrovascular autoregulation. Am J Physiol 1986; 251:H269-281). Mice were anesthetized in preparation for CBF measurement by LDF. After stabilization of MAP and blood gases, MAP was elevated or decreased in 10-mmHg steps, either by intravenous infusion of phenylephrine (1-2 µg · kg⁻¹· min⁻¹) or via controlled exsanguination (100-400 µl of arterial blood), (Ishitsuka T, Iadecola C, Underwood MD, Reis DJ: Lesions of nucleus tractus solitarii globally impair cerebrovascular autoregulation. Am J Physiol 1986; 251:H269-281). The range of MAP studied was 20-160 mmHg. CBF values were recorded 5 min after MAP was changed. In view of potential pathological effects of changes in MAP above or below the autoregulated range, the lower and upper limits of autoregulation were tested in separate animals (Ishitsuka T, Iadecola C, Underwood MD, Reis DJ: Lesions of nucleus tractus solitarii globally impair cerebrovascular autoregulation. Am J Physiol 1986; 251:H269-281).

### Results

### - Measurement of long-term potentiation in hippocampus (CA1/Schaffer collaterals) of Aβ40-injected mice (with or without ARB olmesartan treatment)

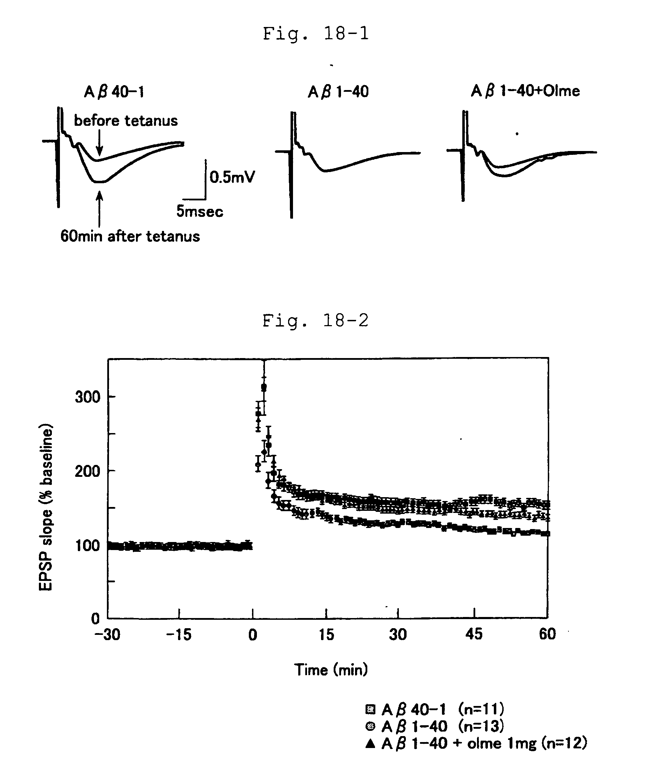

Long-term potentiation (LTP) in the hippocampus is believed to be a cellular basis for learning and memory. The inventors measured long-term potentiation (Fig. 18-1) in the hippocampus of Aβ40-injected mice (with or without ARB olmesartan treatment). ARB olmesartan improved Aβ-induced impairment in spatial memory associated with recovery of LTP (Figs. 18-2 and 18-3).

### - Measurement of cerebral blood flow induced by neuronal activity of Aβ-injected mice (with or without ARB olmesartan treatment)

The inventors measured the cerebral blood flow induced by neuronal activity of Aβ-injected mice (with or without ARB olmesartan treatment). Neuronal activity was evoked in somatosensory-cortex by whisker stimulation (Fig. 19-1). ARB olmesartan improved Aβ-induced impairment in neurovascular coupling (Fig. 19-2).

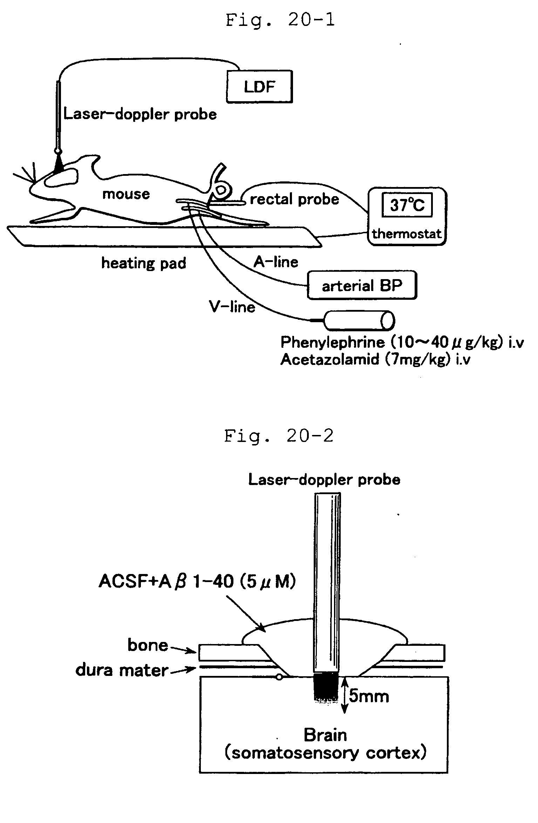

### - Measurement of cerebral blood flow of Aβ-injected mice (with or without ARB olmesartan treatment) (Figs. 20-1 and 20-2)

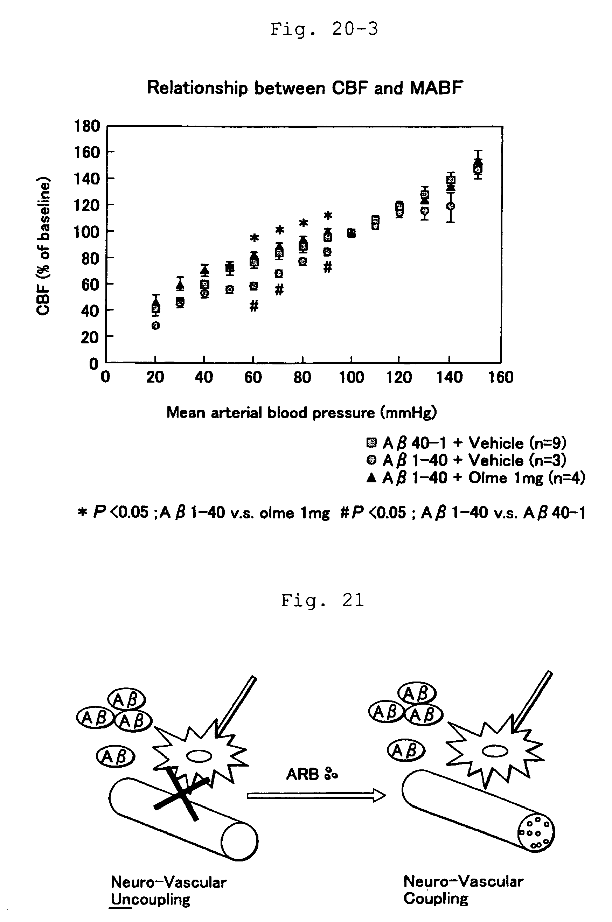

Cerebrovascular autoregulation, i.e., the ability of the cerebral circulation to maintain flow in the face of changes in mean arterial pressure (MAP) is known to be impaired by Aβ. The inventors found cerebrovascular autoregulation impaired by Aβ is recovered by ARB olmesartan (Fig. 20-3).

In summary, it is possible to say that ARB olmesartan recovered Aβ-induced impairment of spatial memory associated with recovery of neurovascular coupling (Fig. 21).

### (FORMULATION EXAMPLE 1) Capsules

| | |
|---|---|
| Olmesartan medoxomil | 20.0 mg |
| Lactose | 158.7 mg |
| Corn starch | 70.0 mg |
| Magnesium stearate | 1.3 mg |
| Total | 250.0 mg |

The above-listed powders are mixed and passed through a 60 mesh screen. The resultant mixed powder is filled in 250 mg No. 3 gelatin capsules.

### (FORMULATION EXAMPLE 2) Tablets

| | |
|---|---|
| Olmesartan medoxomil | 20.0 mg |
| Lactose | 154.0 mg |
| Corn starch | 25.0 mg |
| Magnesium stearate | 1.0 mg |
| Total | 200.0 mg |

The above-listed powders are mixed and pressed on a tabletting machine into tablets each weighing 200 mg. The thus obtained tablets may be coated with sugar, if necessary.

### INDUSTRIAL APPLICABILITY

The medicament of the present invention comprising a specific angiotensin II receptor blocker (such as olmesartan medoxomil) as an active ingredient is useful in treating amyloid β-induce cerebral circulation or cerebral blood flow disorders. This medicament is preferably for use in warm-blooded animals, more preferably for use in human.

Representative causative diseases for dementia are Alzheimer's disease and vascular dementia, but actually a great number of patients with Alzheimer's disease also suffer from cerebrovascular disorder (AD with CVD) (Fig. 17). Recent large-scale epidemiological studies have been elucidating that vascular risk factors typified by hypertension are of independent risk factors of Alzheimer's disease. Hypertension may be a direct modifier of the pathology of Alzheimer's disease. Moreover, hypertension enhances the risk of further decreasing the cognitive function of patients with Alzheimer's disease by causing cerebrovascular disorders. Treatment of hypertension is a crucial element not only in the primary prevention of vascular dementia but also in the management of Alzheimer's disease.

The improvement of cognitive function in Alzheimer's disease model mouse that was achieved by the present invention has suggested that olmesartan is effective in directly improving the pathology of Alzheimer's disease. In actual clinical applications, management of hypertension is further added as an important value. By administering olmesartan to patients with Alzheimer's disease or mild cognitive impairment (MCI) which is an early state of cognitive impairment including Alzheimer's disease and who also suffer from hypertension, it might be possible to inhibit progress of the cognitive impairment. Moreover, by performing hypotensive treatment with olmesartan on hypertensive patients, it might be possible to reduce the risk of the onset of Alzheimer's disease in future. Since management of hypertension is also effective in the prevention of vascular dementia, it is believed that such management may contribute greatly to the overall medical care for dementia.

Specifically, it is contemplated that a currently approved, conventional amount of olmesartan may be administered to hypertension-complicated Alzheimer's disease patients or hypertension-complicated MCI patients to thereby inhibit the progress of cognitive impairment.

## Claims

1. 4-(1-Hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methyl-imidazole-5-carboxylate for use in the treatment of amyloid β-induced cerebral circulation or cerebral blood flow disorders.

2. A composition comprising 4-(1-hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methyl-imidazole-5-carboxylate as an active ingredient, for use in the treatment of amyloid β-induced cerebral circulation or cerebral blood flow disorders.

3. Use of 4-(1-hydroxy-1-methylethyl)-2-propyl-1-[4-[2-(tetrazol-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazol-5-yl)phenyl]phenyl]methylimidazole-5-carboxylate for the manufacture of a medicament for the treatment of amyloid β-induced cerebral circulation or cerebral blood flow disorders.

## Patentansprüche

1. 4-(1-Hydroxy-1-methylethyl)-2-propyl-1-[4-[2-(tetrazol-5-yl)phenyl]phenyl]methylimidazol-5-carbonsäure oder (5-Methyl-2-oxo-1,3-dioxolen-4-yl)methyl-4-(1-hydroxy-1-methylethyl)-2-propyl-1-[4-[2-tetrazol-5-yl)phenyl]phenyl]methylimidazol-5-carboxylat zur Verwendung bei der Behandlung von Amyloid-β-induzierten Störungen des Gehirnkreislaufs oder der Hirndurchblutung.

2. Zusammensetzung, die aufweist:
4-(1-Hydroxy-1-methylethyl)-2-propyl-1-[4-[2-(tetrazol-5-yl)phenyl]phenyl]methylimidazol-5-carbonsäure oder (5-Methyl-2-oxo-1,3-dioxolen-4-yl)methyl-4-(1-hydroxy-1-methylethyl)-2-propyl-1-[4-[2-(tetrazol-5-yl)phenyl]phenyl]methylimidazol-5-carboxylat als Wirkstoff zur Verwendung bei der Behandlung von Amyloid-β-induzierten Störungen des Gehirnkreislaufs oder der Hirndurchblutung.

3. Verwendung von 4-(1-Hydroxy-1-methylethyl)-2-propyl-1-[4-[2-(tetrazol-5-yl)phenyl]phenyl]methylimidazol-5-carbonsäure oder (5-Methyl-2-oxo-1,3-dioxolen-4-yl)methyl-4-(1-hydroxy-1-methylethyl)-2-propyl-1-[4-[2-(tetrazol-5-yl)phenyl]phenyl]methylimidazol-5-carboxylat fiir die Herstellung eines Medikaments zur Behandlung von Amyloid-β-induzierten Störungen des Gehirnkreislaufs oder der Hirndurchblutung.

## Revendications

1. Acide 4-(1-hydroxy-1-méthyléthyl)-2-propyl-1-[4-[2-(tétrazol-5-yl)phényl]phényl]méthyl-imidazole-5-carboxylique ou 4-(1-hydroxy-1-méthyléthyl)-2-propyl-1-[4-[2-(tétrazol-5-yl)phényl]phényl]-méthylimidazole-5-carboxylate de (5-méthyl-2-oxo-1,3-dioxolén-4-yl)méthyle pour une utilisation dans le traitement de troubles de la circulation cérébrale ou du flux sanguin cérébral induits par l'amyloïde β.

2. Composition comprenant:
l'acide 4-(1-hydroxy-1-méthyléthyl)-2-propyl-1-[4-[2-(tétrazol-5-yl)phényl]phényl]méthyl-imidazole-5-carboxylique ou le 4-(1-hydroxy-1-méthyléthyl)-2-propyl-1-[4-[2-(tétrazol-5-yl)phényl]phényl]-méthylimidazole-5-carboxylate de (5-méthyl-2-oxo-1,3-dioxolén-4-yl)méthyle en tant qu'ingrédient actif, pour une utilisation dans le traitement de troubles de la circulation cérébrale ou du flux sanguin cérébral induits par l'amyloïde β.

3. Utilisation de l'acide 4-(1-hydroxy-1-méthyléthyl)-2-propyl-1-[4-[2-(tétrazol-5-yl)phényl]-phényl]méthylimidazole-5-carboxylique ou du 4-(1-hydroxy-1-méthyléthyl)-2-propyl-1-[4-[2-(tétrazol-5-yl)phényl]phényl]méthylimidazole-5-carboxylate de (5-méthyl-2-oxo-1,3-dioxolén-4-yl)méthyle pour la fabrication d'un médicament pour le traitement de troubles de la circulation cérébrale ou du flux sanguin cérébral induits par l'amyloïde β.
